# EUROPEAN PATENT APPLICATION

(11) **EP 3 815 702 A1**
(43) Date of publication of application: **05.05.2021**
(21) Application number: 19206586.0
(22) Date of filing: 31.10.2019
(51) Int. Cl.: A61K 35/745, A61K 35/747, A23L 33/135, A61K 47/10, A61K 47/02, A61K 47/36, A61K 9/14, A61K 9/20

(54) **COMPOSITION FOR PROMOTING GLUCOLIPID METABOLISM, AND PREPARATION AND APPLICATION THEREOF**

(71) Applicant: Ruijin Hospital Shanghai Jiao Tong University School of Medicine, Shanghai (CN); Shanghai Jiao Da Onlly Co., Ltd., Shanghai (CN); Shanghai Novanat Co., Ltd., Shanghai (CN)
(72) Inventor: Ning, Guang, Shanghai (CN); Wang, Weiqing, Shanghai (CN); Gu, Yanyun, Shanghai (CN); Zhang, Yifei, Shanghai (CN); Xia, Yizhi, Shanghai (CN); Jia, Suzhong, Shanghai (CN)
(74) Representative: Schmid, Nils T.F.

(57) **Abstract**

The present invention provides a composition for promoting glucolipid metabolism, and a preparation and an application thereof, and relates to the technical field of probiotics. The composition of the present invention includes probiotics and inulin; the probiotics include *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum,* and *Lactobacillus casei.* In the composition of the present invention, the nine strains synergize with each other, which function as a regulator of glucolipid metabolism, improve sensitivity to insulin receptor in the body, and relieve insulin resistance when reconstituted with inulin. The composition has some effects on improving serum total cholesterol and triglyceride, islet β-cell function, and type 2 diabetes mellitus.

## Description

### TECHNICAL FIELD

The present invention relates to the technical field of probiotics, and in particular to a composition for promoting glucolipid metabolism, and a preparation and an application thereof.

### BACKGROUND

Diabetes mellitus is a chronic metabolic disorder syndrome caused by insulin deficiency, which is manifested mainly by polydipsia, polyuria, polyphagia, and weight loss, and has been the world's third disease seriously threatening human health after cancers and cardio-cerebrovascular diseases to date. Type 2 diabetes mellitus (T2DM) is a group of glucolipid metabolic diseases caused by progressive islet β-cell dysfunction and insulin resistance, which commonly involves in genetic factors and a plurality of environmental factors. The incidence of T2DM increases year by year worldwide, which, in particular, will rise faster and prevail in developing countries. Diabetes mellitus has been the world's third non-infectious disease threatening people's health and lives after cardiovascular diseases and cancers. In T2DM patients, chronic hyperglycemia can be complicated with chronic multiple organ injury, which seriously does harm to patients' health and quality of life. In recent years, with the development of the social economy and the change of people's living styles, in particular high-sugar, high-fat and high-salt diet, the number of diabetics is rising year by year and becoming younger.

So far, oral medication is the main means to treat diabetes mellitus: such as sulfonylureas, glinides, biguanides, thiazolidinediones, and α-glucosidase inhibitors. Moreover, injection of insulin or insulin analogs is an effective way to lower blood sugar as well. However, the foregoing medications are usually restricted in practical use by their clinical defects, such as potential side effects and subordinate invalidation.

### SUMMARY

In view of this, the objective of the present invention is to provide a composition for promoting glucolipid metabolism, which can lower blood lipids, protect islet β-cell function, improve symptoms of type 2 diabetes mellitus (T2DM), and reduce the risk of occurrence and development of diabetes mellitus, featuring safety, reliability, and no side effects.

In order to achieve the foregoing invention objective, the present invention provides the following technical solutions:
The present invention provides a composition for promoting glucolipid metabolism, where the composition includes probiotics and inulin; the probiotics include: *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum,* and *Lactobacillus casei.*

Preferably, the probiotics include the following raw materials in parts by weight: 15-20 parts of *Bifidobacterium longum,* 5-10 parts of *Bifidobacterium breve,* 10-15 parts of *Lactobacillus gasseri,* 10-15 parts of *Lactobacillus rhamnosus,* 10-15 parts of *Lactobacillus salivarius,* 5-10 parts of *Lactobacillus cripatus,* 1-5 parts of *Lactobacillus plantarum,* 1-5 parts of *Lactobacillus fermentum,* and 1-5 parts of *Lactobacillus casei.*

Preferably, all of the raw materials of the probiotics are lyophilized powders, the total count of lactic acid bacteria (LAB) in each of the lyophilized powders is at least 1.0 × 10¹¹ cfu/g, and the total count of lactic acid bacteria (LAB) in the probiotics is at least 1.0 × 10¹¹ cfu/g.

Preferably, the accession number of *Bifidobacterium longum* is CGMCC No.2107;
the accession number of *Bifidobacterium breve* is CGMCC No.6402;
the accession number of *Lactobacillus gasseri* is CGMCC No.10758;
the accession number of *Lactobacillus rhamnosus* is CNCM I-4474;
the accession number of *Lactobacillus salivarius* is CGMCC No.6403;
the accession number of *Lactobacillus cripatus* is CGMCC No.6406;
the accession number of *Lactobacillus plantarum* is CGMCC No.1258;
the accession number of *Lactobacillus fermentum* is CGMCC No.6407; and
the accession number of *Lactobacillus casei* is CNCM I-4458.

The present invention provides a preparation for promoting glucolipid metabolism, including the composition and edible carriers.

Preferably, the edible carriers include one or more of milk powder, maltodextrin, oligosaccharide, dietary fiber, powdered juice, sugar alcohol, starch, magnesium stearate, and silicon dioxide.

Preferably, the total count of lactic acid bacteria (LAB) in the preparation is 1.0-5.0 × 10¹⁰cfu/g.

Preferably, the pharmaceutical dosage form of the preparation comprises powders, tablets, granules, aqueous solutions, pills, capsules, or gels.

The present invention further provides an application of the composition or the preparation in the preparation of food products, pharmaceuticals, or functional food.

Preferably, there is an application of the composition or the preparation in the preparation of food products, pharmaceuticals, or functional food for promoting glucolipid metabolism.

The present invention provides a composition for promoting glucolipid metabolism, where the composition includes probiotics and inulin; the probiotics include: *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum,* and *Lactobacillus casei.* In the composition of the present invention, *Bifidobacterium longum, Bifidobacterium breve, Lactobacillus gasseri, Lactobacillus rhamnosus, Lactobacillus salivarius,* and *Lactobacillus cripatus* are autochthonous probiotics in the gut; after patients with type 2 diabetes mellitus (T2DM) take acarbose orally, the abundance of the foregoing probiotics in the intestinal flora rises significantly and is significantly related to patient's body weight, blood pressure, waist-hip ratio, and glycosylated hemoglobin. Among the probiotics of the present invention, *Lactobacillus plantarum, Lactobacillus fermentum,* and *Lactobacillus casei* can synergically regulate the intestinal flora. In the present invention, the inulin can inhibit the elevation in postprandial blood glucose level, improve sensitivity to insulin receptor in the body, relieve insulin resistance, and further regulate the relevant activity of glucose metabolic enzyme to lower the postprandial blood glucose level. The composition of the present invention combines nine probiotic strains with inulin, which can further regulate the function of glucolipid metabolism. In the embodiments of the present invention, the composition has some effects on improving serum total cholesterol and triglyceride, islet β-cell function, and T2DM, can reduce the risk of occurrence and development of diabetes mellitus, and can be used to prepare food products, pharmaceuticals, or functional food for the prevention and treatment of T2DM.

### Biological depository information

*Bifidobacterium longum* BL88-ONLLY was deposited at China General Microbiological Culture Collection Center (CGMCC) (Institute of Microbiology, Chinese Academy of Sciences, Rendian Road, Chaoyang District, Beijing) on July 13, 2007, with the accession number of CGMCC No. 2107.

*Bifidobacterium breve* BB8 was deposited at China General Microbiological Culture Collection Center (CGMCC) (Institute of Microbiology, Chinese Academy of Sciences, No.3, NO.1 West Beichen Road, Chaoyang District, Beijing) on July 31, 2012, with the accession number of CGMCC No. 6402.

*Lactobacillus gasseri* LG23 was deposited at China General Microbiological Culture Collection Center (CGMCC) (Institute of Microbiology, Chinese Academy of Sciences, No.3, NO.1 West Beichen Road, Chaoyang District, Beijing) on April 28, 2015, with the accession number of CGMCC No. 10758.

*Lactobacillus rhamnosus* LR22 was deposited at the Colección nacional de cultivos de microorganismos (CNCM) (Institut Pasteur, 25 rue du Docteur Roux, F-75724 PARIS Cedex 15, France) on April 26, 2011 under accession number CNCM I-4474.

*Lactobacillus salivarius* LS86 was deposited at China General Microbiological Culture Collection Center (CGMCC) (Institute of Microbiology, Chinese Academy of Sciences, No.3, NO.1 West Beichen Road, Chaoyang District, Beijing) on July 31, 2012, with the accession number of CGMCC No. 6403.

*Lactobacillus cripatus* LCR15 was deposited at China General Microbiological Culture Collection Center (CGMCC) (Institute of Microbiology, Chinese Academy of Sciences, No.3, NO.1 West Beichen Road, Chaoyang District, Beijing) on July 31, 2012, with the accession number of CGMCC No. 6406.

*Lactobacillus plantarum* LP-ONLLY was deposited at China General Microbiological Culture Collection Center (CGMCC) (Institute of Microbiology, Chinese Academy of Sciences, No. 13, Beiyitiao Alley, Zhongguancun, Haidian District, Beijing) on December 6, 2004 with the accession number of CGMCC No. 1258.

*Lactobacillus fermentum* LF33 was deposited at China General Microbiological Culture Collection Center (CGMCC) (Institute of Microbiology, Chinese Academy of Sciences, No.3, NO.1 West Beichen Road, Chaoyang District, Beijing) on July 31, 2012, with the accession number of CGMCC No. 6407.

*Lactobacillus casei* LC18 was deposited at the Coleccion nacional de cultivos de microorganismos (CNCM) (Institut Pasteur, 25 rue du Docteur Roux, F-75724 PARIS Cedex 15, France) on March 28, 2011 under accession number CNCM I-4474.

### BRIEF DESCRIPTION OF THE DRAWINGS

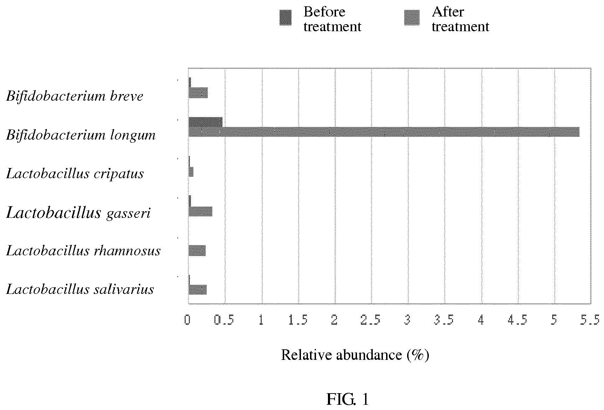

FIG. 1 shows a chart of changes in composition of intestinal flora before and after oral administration of antidiabetic acarbose.

### DETAILED DESCRIPTION

The present invention provides a composition for promoting glucolipid metabolism, where the composition includes probiotics and inulin; the probiotics include: *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum,* and *Lactobacillus casei.*

In the composition of the present invention, the probiotics and the inulin are not particularly limited in the present invention, and any mixing proportions are within the scope of the present invention. In the embodiments of the present invention, the probiotics and the inulin are mixed at a proportion of (58-100):(10-20) parts by weight.

Any proportional relationships of all raw materials in the probiotics are not particularly limited in the present invention, and the foregoing nine probiotics of any mixing proportions are within the scope of the present invention, preferably including the following raw materials in parts by weight: 15-20 parts of *Bifidobacterium longum,* 5-10 parts of *Bifidobacterium breve,* 10-15 parts of *Lactobacillus gasseri,* 10-15 parts of *Lactobacillus rhamnosus,* 10-15 parts of *Lactobacillus salivarius,* 5-10 parts of *Lactobacillus cripatus,* 1-5 parts of *Lactobacillus plantarum,* 1-5 parts of *Lactobacillus fermentum,* and 1-5 parts of *Lactobacillus casei,* and more preferably including: 15-20 parts of *Bifidobacterium longum,* 5-8 parts of *Bifidobacterium breve,* 12-15 parts of *Lactobacillus gasseri,* 10-12 parts of *Lactobacillus rhamnosus,* 10-12 parts of *Lactobacillus salivarius,* 5-8 parts of *Lactobacillus cripatus,* 1-3 parts of *Lactobacillus plantarum,* 1-3 parts of *Lactobacillus fermentum,* and 1-3 parts of *Lactobacillus casei.* In the present invention, the raw materials of the probiotics are preferably lyophilized powders, the total count of lactic acid bacteria (LAB) in each of the lyophilized powders is at least 1.0 × 10¹¹ cfu/g, and the total count of lactic acid bacteria (LAB) in the probiotics is at least 1.0 × 10¹¹ cfu/g. Preparation methods of lyophilized powders of the probiotics are not particularly limited in the present invention, which may be prepared by means of conventional methods in the art; preferably, each probiotic raw material is separately prepared into a lyophilized powder, and then the nine lyophilized powders are mixed to prepare the probiotics. In the present invention, the preparation method of the lyophilized powders preferably includes the following steps of:
1) separately culturing and fermenting the foregoing nine probiotics in MRS broth or modified MRS broth to obtain probiotics-containing fermentation broths;
2) centrifugation: centrifuging and separating each of the foregoing fermentation broths to obtain bacterial sludges; and
3) lyophilization: mixing the bacterial sludges with water (6- to 15-fold the weight of the bacterial sludge) and lyophilisate carriers, and lyophilizing the resulting mixture to prepare into lyophilized powders.

In the present invention, the fermentation temperature is preferably and independently 37-39°C, and more preferably 38°C; the fermentation time is preferably 14-29 h, and more preferably 18-24 h. In the present invention, the viable cell count in each of the preferably obtained fermentation broths is greater than 1.0 × 10⁹cfu/ml, and more preferably greater than 2.0 × 10⁹ cfu/ml.

In the present invention, the centrifugation is carried out in a Class 100000 cleanroom. Centrifugal parameters are not particularly limited in the present invention, and all centrifugal operations that can centrifuge and precipitate viable bacteria from the fermentation broth are within the scope of the present invention.

The lyophilisate carriers of the present invention are preferably one or more of skim milk powder, starch, and maltodextrin. Meanwhile, the skim milk powder further functions as a protector of probiotic activity. Lyophilization parameters are not particularly limited in the present invention, and lyophilization methods known to those skilled in the art may be used.

In the present invention, the probiotics are autochthonous probiotics (*Lactobacillus* sp. and *Bifidobacterium* sp.) in the gut. Moreover, a randomized, double-blind, placebo-controlled clinical study of 106 patients with type 2 diabetes mellitus who received no medication has found after composition analysis of intestinal flora before and after oral administration of acarbose on the premise of significant improvement of glycosylated hemoglobin (HbAic) that acarbose changed the composition of the intestinal flora very significantly while reducing the absorption of glucose in the small intestine. The abundance of some autochthonous probiotics in the gut increases greatly, whereas harmful bacteria decrease significantly in the gut. Of them, two bifidobacteria and four lactobacilli, i.e., *Bifidobacterium longum, Bifidobacterium breve, Lactobacillus gasseri, Lactobacillus rhamnosus, Lactobacillus salivarius,* and *Lactobacillus cripatus,* show a significant increase in abundance (see FIG. 1). It is found that their abundance significantly rises as patient's blood glucose decreases, and is significantly related to patient's body weight, blood pressure, waist-hip ratio, and glycosylated hemoglobin. It follows that these six probiotics with a substantial increase in abundance involve in the function of overall metabolic improvement.

Based on this, the present invention investigates and screens these six probiotics relevant to human metabolic improvement, and along with the other three probiotics which are derived from the intestine, proposed a composition of nine probiotics and inulin promoting glucolipid metabolism. The probiotic raw materials in the probiotics of the present invention are preferably isolated from healthy human intestinal flora, specifically including: using lactobacillus- and Bifidobacterium-selective media to isolate a single colony of the healthy human intestinal flora, identifying and isolating strains by morphology, physiology and biochemistry, and DNA sequencing, followed by *in vitro* probiotic performance evaluation, including: screening of acid resistance, bile salt resistance, drug susceptibility, and fermentation performance, to obtain probiotic strains involved in overall metabolic improvement. Also, the foregoing probiotic species are deposited at patent-specified microbiological culture collection centers, and the specific strains include: *Bifidobacterium longum* BL88-ONLLY (accession number: CGMCC No.2107), *Bifidobacterium breve* BB8 (accession number: CGMCC No.6402), *Lactobacillus gasseri* LG23 (accession number: CGMCC No.10758), *Lactobacillus rhamnosus* LR22 (accession number: CNCM I-4474), *Lactobacillus salivarius* LS86 (accession number: CGMCC No.6403), *Lactobacillus cripatus* LCR15 (accession number: CGMCC No.6406), *Lactobacillus plantarum* LP-ONLLY (accession number: CGMCC No.1258), *Lactobacillus fermentum* LF33 (accession number: CGMCC No.6407), and *Lactobacillus casei* LC18 (accession number: CNCM I-4458).

The composition of the present invention further includes inulin, and the inulin is preferably 10-20 parts by weight. The inulin of the present invention can pass into the large intestine completely without digestion and degradation in the upper digestive tract, which can be decomposed or partially decomposed into short-chain fatty acids (SCFAs) in the large intestine, further influencing the concentration of free fatty acids in the circulation and concentrations of gastrointestinal hormones with glycemic regulation, and thereby improving blood glucose levels; further, inulin can promote the growth of intestinal probiotics, thereby improving intestinal function; moreover, fructo-oligosaccharides derived from inulin play a role in improving blood glucose control and blood lipid metabolism. Sources of the inulin are not particularly limited in the present invention, and conventional inulin in the art may be used.

The present invention provides a preparation for promoting glucolipid metabolism, including the composition and edible carriers.

In the preparation of the present invention, the edible carriers preferably include one or more of milk powder, maltodextrin, oligosaccharide, dietary fiber, powdered juice, sugar alcohol, starch, magnesium stearate, and silicon dioxide. In the preparation of the present invention, the total count of lactic acid bacteria (LAB) is preferably 1.0-5.0 × 10¹⁰cfu/g, more preferably 1.0-4.0 × 10¹⁰ cfu/g, and most preferably 2.0-3.0 × 10¹⁰ cfu/g.

The pharmaceutical dosage form of the preparation is not particularly limited in the present invention, and preferably includes powders, tablets, granules, aqueous solutions, pills, capsules, or gels. In the present invention, when preparing the preparation into different pharmaceutical dosage forms, additives required to prepare different preparations can also be added preferably, e.g., wetting agent, disintegrant, dispersant, emulsifier, and so forth, which can be added properly by those skilled in the art according to the requirements for different pharmaceutical dosage forms; conventional preparation methods in the art may be used.

The present invention further provides an application of the composition or the preparation in the preparation of food products, pharmaceuticals, or functional food. The application of the present invention is preferably an application of preparation of food products, pharmaceuticals, or functional food for promoting glucolipid metabolism.

A composition for promoting glucolipid metabolism, and a preparation and an application thereof of the present invention will be described in detail below in conjunction with the embodiments, but they should not be construed as a limitation to the scope of the invention.

### Embodiment 1

Nine probiotics and inulin, weighing as follows, were mixed and prepared into a composition: 20 g of *Bifidobacterium longum,* 10 g of *Bifidobacterium breve,* 15 g of *Lactobacillus gasseri,* 15 g of *Lactobacillus rhamnosus,* 15 g of *Lactobacillus salivarius,* 5 g of *Lactobacillus cripatus,* 3 g of *Lactobacillus plantarum,* 3 g of *Lactobacillus fermentum,* 3 g of *Lactobacillus casei,* and 11 g of inulin. In the composition prepared, the total count of lactic acid bacteria (LAB) was not less than 1.0 × 10¹¹ cfu/g.

### Embodiment 2

Nine probiotics and inulin, weighing as follows, were mixed and prepared into a composition: 15 g of *Bifidobacterium longum,* 10 g of *Bifidobacterium breve,* 12 g of *Lactobacillus gasseri,* 15 g of *Lactobacillus rhamnosus,* 14 g of *Lactobacillus salivarius,* 8 g of *Lactobacillus cripatus,* 2 g of *Lactobacillus plantarum,* 2 g of *Lactobacillus fermentum,* 2 g of *Lactobacillus casei,* and 20 g of inulin. In the composition prepared, the total count of lactic acid bacteria(LAB) was not less than 2.0 × 10¹¹ cfu/g.

### Embodiment 3

Nine probiotics and inulin, weighing as follows, were mixed and prepared into a composition: 19 g of *Bifidobacterium longum,* 8 g of *Bifidobacterium breve,* 13 g of *Lactobacillus gasseri,* 10 g of *Lactobacillus rhamnosus,* 13 g of *Lactobacillus salivarius,* 9 g of *Lactobacillus cripatus,* 2 g of *Lactobacillus plantarum,* 3 g of *Lactobacillus fermentum,* 3 g of *Lactobacillus casei,* and 20 g of inulin. In the composition prepared, the total count of lactic acid bacteria (LAB) was not less than 3.0 × 10¹¹ cfu/g.

### Embodiment 4

Nine probiotics and inulin, weighing as follows, were mixed and prepared into a composition: 18 g of *Bifidobacterium longum,* 9 g of *Bifidobacterium breve,* 10 g of *Lactobacillus gasseri,* 10 g of *Lactobacillus rhamnosus,* 10 g of *Lactobacillus salivarius,* 10 g of *Lactobacillus cripatus,* 4 g of *Lactobacillus plantarum,* 5 g of *Lactobacillus fermentum,* 5 g of *Lactobacillus casei,* and 19 g of inulin. In the composition prepared,the total count of lactic acid bacteria (LAB) was not less than 4.0 × 10¹¹ cfu/g.

### Embodiment 5

The probiotic composition of Embodiment 1 was mixed well with 500 g of galacto-oligosaccharide, 150 g of maltodextrin, 120 g of strawberry powders, and 30 g of sorbitol to prepare probiotic powders.The total count of lactic acid bacteria (LAB) in the product was not less than 1.0 × 10¹⁰ cfu/g.

### Embodiment 6

The composition of Embodiment 2 was mixed with 400 g of xylo-oligosaccharides, 200g of dietary fiber, 100g of starch and pelletized, the granules was mixed with 10g magnesium stearate, and tableted into probiotics tablets. The total count of lactic acid bacteria (LAB) in the product was not less than 2.0 × 10¹⁰ cfu/g.

### Embodiment 7

The composition of Embodiment 3 was mixed well with 520 g of resistant dextrin to prepare probiotic powders. The total count of lactic acid bacteria (LAB) in the product was not less than 3.0 × 10¹⁰ cfu/g.

### Embodiment 8

The composition of Embodiment 4 was mixed with 580 g of milk powder to prepare probiotics-containing milk powder. The total count of lactic acid bacteria (LAB) in the product was not less than 4.0 × 10¹⁰ cfu/g.

### Embodiment 9

### Effect of composition of probiotics and inulin on blood glucose in type 2 diabetic mice

High-glucose-high-fat diet plus streptozotocin (STZ) was induced to establish type 2 diabetic mouse models, which were divided into two groups of 11: a control group and a probiotics/inulin composition group (hereinafter referred to as probiotic composition group). The composition group was given the composition of nine probiotics and inulin of Embodiment 1, i.e., including *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum, Lactobacillus casei,* and inulin, by gavage daily, and total lactobacillus count was 10⁹ for each mouse. The control group was given normal saline by gavage. Both groups were administered for 24 days. Random blood glucose was tested by glucometer.

**Table 1 Effect of composition of probiotics and inulin on random blood glucose in type 2 diabetic model mice**

| Groups | Blood glucose level, in mmol/L | |
|---|---|---|
| | Before experiment | After experiment |
| Control Group | 27.95 ± 3.64 | 28.65 ± 3.56 |
| Probiotic Composition Group | 29.12 ± 4.45 | 23.93 ± 2.46* |
| NOTE: *, significantly different from the control group, *P<*0.05*.* | | |

As seen from Table 1, the composition of probiotics and inulin of the present invention can significantly improve blood glucose levels in type 2 diabetic model mice and has a hypoglycemic effect.

Existing research (Zhong D, Yin Y, Ge M, Qian XP. Study on the reduction of postprandial blood glucose with acarbose promoted by four-combination probiotics in diabetic mice[J]. Chin Med Biotechnol, 2016,11(5):441-444) reported that administration of four-combination probiotics (*Lactobacillus acidophilus, Lactobacillus rhamnosus, Bifidobacterium longum,* and *Bacillus licheniformis*) alone in diabetic mice had no significant effect on reduction of postprandial blood glucose. However, the composition of probiotics and inulin of the present invention can significantly improve blood glucose levels in diabetic model mice.

### Embodiment 10

### Effects of composition of probiotics and inulin on serum cholesterol and triglyceride in type 2 diabetic mice

High-glucose-high-fat diet plus streptozotocin (STZ) was induced to establish type 2 diabetic mouse models, which were divided into two groups of 11: a control group and a probiotic composition group. The probiotic composition group was given the composition of nine probiotics and inulin of Embodiment 1, i.e., including *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum, Lactobacillus casei,* and inulin, by gavage daily, and total lactobacillus count was 10⁹ for each mouse. The control group was given normal saline by gavage. Both groups were administered for 24 days. Mouse serum cholesterol and triglyceride levels were tested by dry biochemical analyzer.

**Table 2 Effect of composition of probiotics and inulin on blood lipids in type 2 diabetic model mice**

| Groups | Control Group | Probiotic Composition Group |
|---|---|---|
| Serum cholesterol, mmol/L | 5.975 ± 0.4437 | 5.059±0.2118a |
| Triglyceride, mmol/L | 0.932 ± 0.0894 | 0.6042±0.07383b |
| NOTE: "a", significantly different from the control group, *P<*0.05 | | |
| "b", different from the control group, *P*=0.0548 | | |

As seen from Table 2, the probiotic composition of the present invention can significantly lower cholesterol in type 2 diabetic model mice and improve triglyceride levels.

Type 2 diabetes mellitus (T2DM) is usually accompanied by dyslipidemia. Hyperlipidemia is an important factor causing the development of atherosclerosis. Therefore, the composition of probiotics and inulin of the present invention has hypolipidemic effect, thereby relieving the risk of development of cardiovascular complications in those with T2DM.

### Embodiment 11

### Effect of probiotic composition on postprandial serum C-peptide in diabetic mice

High-glucose-high-fat diet plus streptozotocin (STZ) was induced to establish type 2 diabetic mouse models, which were divided into two groups of 11: a control group and a probiotic composition group. The probiotic composition group was given the composition of nine probiotics and inulin of Embodiment 1, i.e., including *Bifidobacterium longum, Bifidobacterium breve,Lactobacillus gasseri, Lactobacillus rhamnosus, Lactobacillus salivarius, Lactobacillus cripatus, Lactobacillus plantarum, Lactobacillus fermentum, Lactobacillus casei,* and inulin, by gavage daily, and the total count of lactic acid bacteria (LAB) was 10⁹ for each mouse by gavage daily. The control group was given normal saline by gavage. Both groups were administered for 24 days. Postprandial 30 min serum C-peptide was assayed by ELISA.

**Table 3 Effect of composition of probiotics and inulin on postprandial serum C-peptide in type 2 diabetic model mice**

| Groups | Serum C-peptide, pg/ml | | |
|---|---|---|---|
| | 0 min | 15 min | 30 min |
| Control Group | 5487 ± 4448 | 5615 ± 4649 | 3003 ± 1573 |
| Probiotic Composition Group | 5385 ± 1764 | 7678 ± 2560 | 5204 ± 1269* |
| NOTE: *, significantly different from the control group, *P<*0.05*.* | | | |

Serum C-peptide is a secretory product of islet β-cell, and its detection value can directly reflect islet β-cell function; if the islet β-cell function is impaired, disturbance of glucose metabolism will occur, resulting in an abnormal rise in blood glucose. As seen from Table 3, the probiotic composition group can significantly improve postprandial 30 min serum C-peptide values in diabetic model mice, indicating that the composition of probiotics and inulin of the present invention plays a certain role in recovering islet β-cell function impaired by diabetes mellitus.

### Embodiment 12

### Effect of composition of probiotics and inulin on postprandial 2 hr insulin in patients with new-onset type 2 diabetes mellitus (T2DM)

Using the composition of nine probiotics and inulin of Embodiment 7, i.e., including *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum, Lactobacillus casei,* and inulin, with a total count of lactic acid bacteria (LAB) of 3.0 × 10¹⁰ cfu/g, a double-blind, placebo-controlled, population-based trial was conducted on patients with new-onset type 2 diabetes mellitus (T2DM). The placebo was maltodextrin, with identical packaging.

A total of 30 eligible patients with new-onset T2DM were selected. Each group included 15 patients who took 4 g once a day. For the experimental group, 1.2 × 10¹¹ viable bacteria were administered daily, for 12 weeks. Before the screening period, blood glucose was only controlled by life style intervention (diet and sports) for at least two months; medications were not used for blood glucose control; during the screening period, glycosylated hemoglobin A1c (HbAic) ranged from 6.5% to 10.0%, while fasting plasma glucose (FPG) values were between 7.0 mmol/L and 13.3 mmol/L on the day of screening.

**Table 4 Effect of composition of probiotics and inulin on postprandial 2 hr insulin in patients with new-onset T2DM**

| Groups | Postprandial 2 hr insulin, IU/ml | | |
|---|---|---|---|
| | Before administration | After administration | Difference (pre-experimental vs. post-experimental) |
| Placebo Group | 60.12 ± 26.24 | 48.79 ± 13.86 | 11.33 ± 8.52 |
| Probiotic Composition Group | 43.38 ± 25.66 | 49.87 ± 25.94 | -6.49 ± 6.11* |
| NOTE: *, significantly different from the control group, *P<*0.05*.* | | | |

Like C-peptide, serum insulin is also a secretory product of islet β-cell, and its detection value can directly reflect islet β-cell function; if the islet β-cell function is impaired, disturbance of glucose metabolism will occur, resulting in an abnormal rise in blood glucose. As seen from Table 4, the probiotic composition group can significantly improve difference in postprandial 2 hr insulin, indicating that the probiotic composition of the present invention plays a certain role in recovering islet β-cell function impaired by diabetes mellitus.

### Embodiment 13

### Composition of probiotics and inulin in the promotion of glucolipid metabolism

An adult male volunteer diagnosed with new-onset type 2 diabetes mellitus (T2DM), who did not use any medication for blood glucose control, took 4 g of probiotic powders of Embodiment 7 of the present invention (1.2 × 10¹¹ viable bacteria) once a day, for 12 weeks. Glucolipid metabolism-related indexes were tested before and after administration. Results are shown in Table 5.

**Table 5 Composition of probiotics and inulin promotes indexes related to glucolipid metabolism**

| Indexes | Before administration | After administration | Description: |
|---|---|---|---|
| Body mass index BMI, Kg/m² | 28.14 | 25.17 | Decreased by 10.55% |
| Glycosylated hemoglobin HbA1c, % | 6.71 | 5.82 | Decreased by 13.26% |
| Fasting plasma glucose FPG, mmol/L | 7.75 | 6.59 | Decreased by 14.97% |
| Serum cholesterol TC, mmol/L | 6.71 | 6.52 | Decreased by 2.8% |
| Triglyceride TG, mmol/L | 2.66 | 2.02 | Decreased by 24.06% |
| Postprandial 2 hr insulin | 39.92 | 50.21 | Increased by 25.78% |

As seen from Table 5, after the patient with new-onset T2DM took the composition of the present invention for 12 weeks, his indexes related to blood glucose, including HbAic and FPG, decreased by 13.26% and 14.97%, respectively; in indexes related to blood lipids, TG decreased by >20%, and TC improved slightly and decreased by 2.8%; postprandial 2 hr insulin increased, suggesting that the composition recovers islet β-cell function impaired by diabetes mellitus to some extent.

The foregoing embodiments further support that the composition of probiotics and inulin of the present invention promotes glucolipid metabolism, lowers blood glucose, significantly reduces blood lipids, recovers islet β-cell function impaired by diabetes mellitus to some extent, improves diabetic symptoms, and reduces the risk of occurrence and development of diabetes mellitus. It should be noted that, because probiotics containing in the composition of the present invention is a dynamic process in the gut after administration, the difference in proportion of nine probiotics and inulin containing in the composition of the present invention is of no significance to its regulatory function of glucolipid metabolism, and all combinations including the nine probiotics and the inulin can achieve a considerable role in regulating glucolipid metabolism.

The foregoing descriptions are only preferred implementation manners of the present invention. It should be noted that for a person of ordinary skill in the art, several improvements and modifications may further be made without departing from the principle of the present invention. These improvements and modifications should also be deemed as falling within the protection scope of the present invention.

## Claims

1. A composition for promoting glucolipid metabolism, wherein the composition comprises probiotics and inulin; the probiotics comprise: *Bifidobacterium longum, Bifidobacterium breve, Lactobacillus gasseri, Lactobacillus rhamnosus, Lactobacillus salivarius, Lactobacillus cripatus, Lactobacillus plantarum, Lactobacillus fermentum,* and *Lactobacillus casei.*

2. The composition according to claim 1, wherein the probiotics comprise the following raw materials in parts by weight: 15-20 parts of *Bifidobacterium longum,* 5-10 parts of *Bifidobacterium breve,* 10-15 parts of *Lactobacillus gasseri,* 10-15 parts of *Lactobacillus rhamnosus,* 10-15 parts of *Lactobacillus salivarius,* 5-10 parts of *Lactobacillus cripatus,* 1-5 parts of *Lactobacillus plantarum,* 1-5 parts of *Lactobacillus fermentum,* and 1-5 parts of *Lactobacillus casei.*

3. The composition according to claim 1 or 2, wherein all of the raw materials of the probiotics are lyophilized powders, the total count of lactic acid bacteria (LAB) in each of the lyophilized powders is at least 1.0 × 10¹¹ cfu/g, and the total count of lactic acid bacteria (LAB) in the probiotics is at least 1.0 × 10¹¹ cfu/g.

4. The composition according to claim 1, 2 or 3, wherein the accession number of *Bifidobacterium longum* is CGMCC No.2107;
the accession number of *Bifidobacterium breve* is CGMCC No.6402;
the accession number of *Lactobacillus gasseri* is CGMCC No. 10758;
the accession number of *Lactobacillus rhamnosus* is CNCM I-4474;
the accession number of *Lactobacillus salivarius* is CGMCC No.6403;
the accession number of *Lactobacillus cripatus* is CGMCC No.6406;
the accession number of *Lactobacillus plantarum* is CGMCC No.1258;
the accession number of *Lactobacillus fermentum* is CGMCC No.6407; and
the accession number of *Lactobacillus casei* is CNCM I-4458.

5. A preparation for promoting glucolipid metabolism, comprising the composition according to any of claims 1 to 4 and edible carriers.

6. The preparation according to claim 5, wherein the edible carriers comprise one or more of milk powder, maltodextrin, oligosaccharide, dietary fiber, powdered juice, sugar alcohol, starch, magnesium stearate, and silicon dioxide.

7. The preparation according to claim 5 or 6, wherein the total count of lactic acid bacteria (LAB) in the preparation is 1.0-5.0 × 10¹⁰ cfu/g.

8. The preparation according to any of claims 5 to 7, wherein the pharmaceutical dosage form of the preparation comprises powders, tablets, granules, aqueous solutions, pills, capsules, or gels.

9. An application of the composition according to any of claims 1 to 4 or the preparation according to any of claims 5 to 8 in the preparation of food products, pharmaceuticals, or functional food.

10. The application according to claim 9, wherein there is an application of the composition or the preparation in the preparation of food products, pharmaceuticals, or functional food for promoting glucolipid metabolism.
